# EUROPEAN PATENT SPECIFICATION

(11) **EP 3 513 825 B1**
(45) Date of publication and mention of the grant of the patent: **25.08.2021**
(21) Application number: 17851001.2
(22) Date of filing: 15.09.2017
(51) Int. Cl.: A61M 5/315

(54) **SYRINGE PLUNGER, PRESSURIZATION DEVICE FOR SYRINGES, AND SYRINGE SYSTEM**
SPRITZENKOLBEN, DRUCKBEAUFSCHLAGUNGSVORRICHTUNG FÜR SPRITZEN UND SPRITZENSYSTEM
PISTON DE SERINGUE, DISPOSITIF DE MISE SOUS PRESSION POUR SERINGUES ET SYSTÈME DE SERINGUE

(30) Priority: 16.09.2016 JP 2016181519; 16.09.2016 JP 2016181537; 16.09.2016 JP 2016181554
(43) Date of publication of application: 24.07.2019
(73) Proprietor: Terumo Kabushiki Kaisha, Tokyo 151-0072 (JP)
(72) Inventor: TAKAHASHI, Hiroaki, Kanagawa 259-0151 (JP); OKIHARA, Hitoshi, Fujinomiya-shi Shizuoka 418-0004 (JP)
(74) Representative: Casalonga
(86) International application number: PCT/JP2017/033375
(87) International publication number: WO 2018/052101

(56) References cited:
- WO-A1-2009/128393
- WO-A1-2012/173553
- JP-A- H11 182 524
- JP-A- 2007 530 235
- JP-A- 2008 167 954
- JP-U- H0 522 825
- KR-B1- 101 255 970

## Description

### BACKGROUND

### Technical Field

The present invention relates to a syringe pressurization device to be attached to a syringe having a barrel and a gasket sliding inside the barrel.

### Related Art

In related art, a so-called threaded syringe configured to advance a plunger by a screwing action of a screw in order to discharge a drug solution having a relatively high viscosity from a syringe barrel has been known (for example, see JP 2005-535415 A). The screw threaded syringe of JP 2005-535415 A includes a syringe barrel, a grip (a sliding part 14) and a plunger. The grip is a member attached to a flange of the syringe barrel, and includes a hole with a male screw. A male screw is disposed on an outer circumferential surface of the shaft of the plunger. At a proximal end portion of the plunger, a large-diameter handle protruding outward is disposed as a rotary operation part.

JP 2007 530235A discloses a syringe plunger configured to be attached to a syringe including a barrel and a gasket sliding inside the barrel, the syringe plunger comprising:
a plunger rod 14 including a screw thread disposed on an outer circumferential surface thereof; and
a rotary operation part 12 disposed at a proximal end portion of the plunger rod,
the screw thread is configured to be screwed with a female screwing part 16 attached to the syringe,
the syringe plunger is configured to advance in a distal end direction by a screwing action between the screw thread and the female screwing part so as to press the gasket in a distal end direction.

JP H05 22825 U and JP H11 182524 A each show a rotary operation part that includes a finger hook rotary operation part protruding in a direction perpendicular to an axis of the plunger rod, and a pinch rotary operation part protruding in a proximal end direction from the finger hook rotary operation part coaxially with the plunger rod, whereby an outer diameter of the pinch rotary operation part is smaller than an outer diameter of the finger hook rotary operation part.

### SUMMARY

In JP 2005-535415 A, although a large torque can be applied by operating a large-diameter handle disposed at the proximal end portion of the plunger, it is difficult to quickly rotate and advance the plunger.

The present invention has been made in view of such problems, and an object of the present invention is to provide a syringe pressurization device configured to be attached to a syringe system capable of selecting application of a large torque to a syringe plunger and quick rotation and advancement of the syringe plunger, depending on the situation.

To achieve the aforementioned object, the present invention provides a syringe pressurization device according to independent claim 1 The dependent claims relate to advantageous embodiments.

According to the present invention, when it is not necessary to apply a large torque to the plunger, by operating the pinch rotary operation part, the plunger can be rotated quickly and the plunger can be advanced quickly. Therefore, the treatment using the syringe can be efficiently performed.

In the aforementioned syringe plunger, the finger hook rotary operation part may protrude rather than the screw thread in a direction perpendicular to the axis of the plunger rod.

With this configuration, a larger torque can be applied to the syringe plunger.

In the aforementioned syringe plunger, the outer diameter of the pinch rotary operation part may be smaller than the outer diameter of the screw thread.

With this configuration, it is easy to rotate the plunger more quickly when pinching and operating the pinch rotary operation part.

In the aforementioned syringe plunger, the pinch rotary operation part may have a cylindrical shape.

With this configuration, it is easy to rotate the plunger more quickly when pinching and operating the pinch rotary operation part.

In the aforementioned syringe plunger, an anti-slip structure for a finger may be disposed on an outer circumferential portion of the pinch rotary operation part.

With this configuration, slippage of the finger is suppressed when the pinch rotary operation part is operated by being pinched and the operation is easily performed.

In the aforementioned syringe plunger, the finger hook rotary operation part may have a pair of rod-like finger hook parts protruding in directions opposite to each other from the plunger rod.

With this configuration, it is easy to apply a large torque to the syringe plunger when operating the finger hook rotary operation part by hooking a finger.

In the syringe plunger, a constricted part, and a bulging part disposed outside the constricted part may be disposed in the pair of rod-like finger hook parts.

With this configuration, when operating the finger hook rotary operation part by hooking a finger, the user can easily hook his/her fingers.

According to the syringe pressurization device of the present invention, when rotated in the forward direction for advancement of the plunger, the displacement mechanism part disposed in the grip is repelled by the pressing protrusion disposed on the screw thread of the plunger. Further, the displacement mechanism part repelled by the pressing protrusion is displaced by the elastic restoring force and collides against the wall disposed on the grip. Therefore, a click sound having a constant magnitude can be generated, irrespective of the rotational speed of the plunger. Thus, even when the plunger is slowly rotated, the click sound can be reliably generated.

In the aforementioned syringe pressurization device, the displacement mechanism part may be displaced substantially along a direction of the tangential force acting on the pressing protrusion which starts to press the displacement mechanism part at the time of the forward rotation of the plunger rod.

With this configuration, since the force received by the displacement mechanism part from the pressing protrusion increases, and the displacement amount of the displacement mechanism part also increases, a larger click sound can be generated.

In the aforementioned syringe pressurization device, the grip may have a support part supporting the displacement mechanism part, and the displacement mechanism part may have an elastic piece part extending from the support part.

Thus, elastic energy can be accumulated when pressed by the pressing protrusion with a simple structure.

In the aforementioned syringe pressurization device, the elastic piece part may have a U-shaped curved part which opens in a direction substantially opposite to a direction of a tangential force acting on the pressing protrusion which starts to press the displacement mechanism part at the time of the forward rotation of the plunger rod.

Since this makes it easy to balance well the easiness of deformation of the displacement mechanism part and the strength of the elastic restoring force, it is possible to generate a clear click sound, while suppressing an increase in the rotation resistance of the plunger.

In the aforementioned syringe pressurization device, the grip may have an internal space in which the displacement mechanism part is disposed and that echoes a sound generated with collision of the displacement mechanism part to the wall.

Therefore, it is possible to amplify a sound pressure level of the click sound generated by the collision of the displacement mechanism part to the wall in the space, and to provide the user with a clear click sound.

In the aforementioned syringe pressurization device, a pressed part to be pressed by the pressing protrusion at the time of the forward rotation of the plunger rod may be disposed on the displacement mechanism part, and a rod position restricting part configured to restrict a position of the plunger rod with respect to the pressed part on a side opposite to the pressed part on the basis of the plunger rod may be disposed at the grip.

With this configuration, when the plunger rod rotates, it is possible to reliably press the pressed part in the circumferential direction by the pressing protrusion.

In the aforementioned syringe pressurization device, at the time of a reverse rotation of the plunger for a backward movement in a proximal end direction, the displacement mechanism part may be pressed by the pressing protrusion so as climb over the pressing protrusion with a displacement in a direction different from the displacement direction of the displacement mechanism part at the time of the forward rotation of the plunger.

With this configuration, since the reverse rotation of the plunger is not inhibited by the pressing protrusion (the plunger idles in relation to the pressing protrusion), the plunger is rotated in the reverse direction to release the pressure inside the syringe, and the discharge of the drug solution can be stopped. Further, when the plunger rotates in the reverse direction, since the displacement mechanism part does not collide against the wall, the plunger can be idly rotated without generating a clear sound like a click sound.

In the aforementioned syringe pressurization device, the grip may have a support part supporting the displacement mechanism part, the displacement mechanism part may have an elastic piece part extending from the support part, and a pressed part pressed by the pressing protrusion at the time of the forward rotation of the plunger rod, and the elastic piece part may extend from the support part along a direction in which the pressed part is pressed and displaced at the time of the forward rotation of the plunger rod.

With this configuration, at the time of reverse rotation of the plunger, the displacement mechanism part is likely to deform radially outward of the plunger along the wall. Therefore, the plunger can be rotated in the reverse direction without applying a large torque.

In the aforementioned syringe pressurization device, the elastic piece part may have a first extending part extending from the support part along a direction in which the pressed part is pressed and displaced at the time of the forward rotation of the plunger rod, a curved extending part extending from an extending distal end of the first extending part with being curved in a direction opposite to the extending direction of the first extending part, and a second extending part extending from the extending distal end of the curved extending part in the direction opposite to the extending direction of the first extending part, and the pressed part may be disposed at the extending distal end of the second extending part.

Further, a syringe system used in the present invention includes the aforementioned syringe pressurization device, the syringe, and a drug solution filled in a liquid chamber formed by the barrel and the gasket of the syringe.

The present invention provides a syringe pressurization device attached to a syringe having a barrel and a gasket sliding inside the barrel, the syringe pressurization device including: a plunger configured to advance the gasket in a distal end direction; and a grip attachable to the barrel from a direction perpendicular to an axis of the barrel and supporting the plunger, in which the plunger includes a plunger rod including a screw thread disposed on an outer circumferential surface thereof, and a pressing part disposed at a distal end of the plunger rod and configured to press the gasket in a distal end direction, the grip includes a flange attachment part attachable to a flange disposed at a proximal end portion of the barrel, a female screwing part disposed on proximal of the flange attachment part and screwed to the screw thread, and an accommodation part disposed between the flange attachment part and the female screwing part and configured to accommodate the pressing part.

According to the syringe pressurization device of the present invention having the aforementioned configuration, the accommodation part capable of accommodating the pressing part of the plunger is disposed between the flange attachment part and the female screwing part of the grip. Therefore, the syringe pressurization device in a state in which the grip and the plunger are assembled can be attached to the barrel from a direction perpendicular to the axis of the barrel. Therefore, one-touch attachment of the syringe pressurization device to the syringe is possible.

In the aforementioned syringe pressurization device, the plunger rod may have a rotation support part at a distal end portion thereof, the pressing part is a disc-shaped member supported on the rotation support part of the plunger rod to be rotatable about the axis of the plunger rod and having a larger diameter than that of the plunger rod, and the accommodation part is a circular groove recessed in the proximal end direction with respect to the flange attachment part and having an inner diameter larger than an outer diameter of the pressing part.

According to this configuration, it is possible to secure liquid-tightness between the inner surface of the barrel and the gasket by preventing rotation of the gasket with rotation of the plunger, while realizing one-touch attachment by disposing the accommodation part.

In the aforementioned syringe pressurization device, the rotation support part may have a locking claw protruding outward at a distal end thereof, the pressing part may have a tubular base part constituting a center side thereof, and a ring-shaped abutting part extending outward from the tubular base part and configured to abut on the proximal end surface of the gasket, a ring-shaped locking protrusion protruding inward may be disposed on an inner circumferential surface of the tubular base part, and the pressing part may be prevented from slipping out from the rotation support part in the distal end direction, by the locking claw and the ring-shaped locking protrusion.

According to this configuration, the rotatably coupled state between the pressing part and the plunger rod is satisfactorily maintained.

In the aforementioned syringe pressurization device, in a state in which the pressing part is accommodated deepest with respect to the accommodation part, a distal end surface of the pressing part may be located at the proximal end side of the most distal end position of the accommodation part.

According to this configuration, when the syringe pressurization device is attached to the barrel, it is possible to reliably prevent the pressing part from being caught by the proximal end portion of the barrel.

Further, a syringe system according used in the present invention includes a prefilled syringe including a barrel, a gasket disposed to be slidable in the barrel, and a drug solution filled in a liquid chamber formed by the barrel and the gasket, and a syringe pressurization device attachable to the prefilled syringe, in which the syringe pressurization device is the syringe pressurization device according to one of the aforementioned aspects.

Thus, the syringe pressurization device that can be attached with one touch is particularly useful when applied to a pre-filled syringe.

In the aforementioned syringe system, the pressing part may be rotatable about an axis of the plunger rod with respect to the plunger rod, and frictional resistance between the pressing part and the gasket generated when the pressing part rotates about the axis of the plunger rod with respect to the gasket may be greater than frictional resistance between the pressing part and the plunger rod generated when the pressing part rotates about the axis of the plunger rod with respect to the plunger rod.

According to this configuration, when the gasket is pressed with the rotation of the plunger, the gasket advances without rotating inside the barrel. Therefore, deformation of the gasket can be suppressed, and the liquid-tightness between the inner surface of the barrel and the gasket can be favorably secured.

### BRIEF DESCRIPTION OF DRAWINGS

FIG. 1 is a perspective view of a syringe system including a syringe and a syringe pressurization device;
FIG. 2 is an exploded perspective view of the syringe pressurization device;
FIG. 3 is a cross-sectional view along an axis of the syringe pressurization device;
FIG. 4 is a cross-sectional view perpendicular to the axis of the syringe pressurization device;
FIG. 5 is a cross-sectional view of a main part of the syringe pressurization device;
FIG. 6 is a perspective cross-sectional view from a proximal end side of a first member;
FIG. 7 is a perspective view of an assembled state of the syringe and the syringe pressurization device;
FIG. 8 is a cross-sectional view of a state in which a gasket is advanced by a plunger;
FIG. 9A is an explanatory view of a state in which a pressing protrusion of the plunger that rotates in a forward direction moves a displacement mechanism part of a grip, and FIG. 9B is an explanatory view of a state in which the displacement mechanism part repelled by the pressing protrusion collides against a wall of the grip;
FIG. 10 is an explanatory view of a state in which the two pressing protrusions of the plunger abut on the displacement mechanism part of the grip;
FIG. 11A is an explanatory view of a state in which the displacement mechanism part gets on the pressing protrusion of the plunger that rotates in a reverse direction, and FIG. 11B is an explanatory view of a state in which the displacement mechanism part is disengaged from the pressing protrusion of the plunger that rotates in the reverse direction;
FIG. 12 is an explanatory view of a state in which the syringe is connected to a proximal end portion of a puncturing device and a needle disposed at a distal end portion of an inner catheter of the puncturing device is caused to protrude from the distal end of an outer catheter; and
FIG. 13A is an explanatory view of a process of causing the needle of the puncturing device to protrude from a distal end of an endoscope in a living body, FIG. 13B is an explanatory view of a process of injecting hyaluronic acid into a tissue under the lesioned part to raise the lesioned part, FIG. 13C is an explanatory view of a process of cutting a mucous membrane around the lesioned part by a peeling device, and FIG. 13D is an explanatory view of a process of collecting the mucous membrane having the lesioned part by a gripping device.

### DETAILED DESCRIPTION

Preferred embodiments of the present invention will be described below with reference to the accompanying drawings.

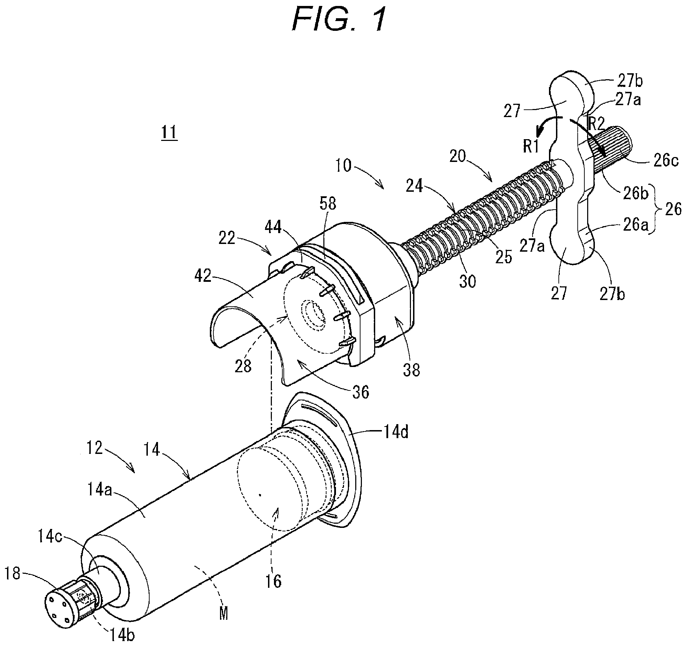

A syringe pressurization device 10 (hereinafter abbreviated as a "pressurization device 10") illustrated in FIG. 1 is a device which is attached to a syringe 12 and is used for discharging a drug solution M in the syringe 12 by pressing a gasket 16 of the syringe 12 in a distal end direction. The syringe system 11 is constituted by the syringe 12 and the pressurization device 10.

In FIG. 1, the syringe 12 includes a hollow tubular barrel 14, a gasket 16 (also referred to as a stopper) disposed inside the barrel 14 to be axially slidable in a liquid-tight manner, and the drug solution M filled in a liquid chamber formed by the barrel 14 and the gasket 16. That is, the syringe 12 is configured as a prefilled syringe in which the drug solution M is prefilled in the barrel 14.

The drug solution M has a relatively high viscosity such that the pressurization device 10 is required for discharging from the syringe 12, and for example, a sodium hyaluronate solution can be used. The viscosity of the drug solution M is, for example, in a range of 50 to 120 mPa·s.

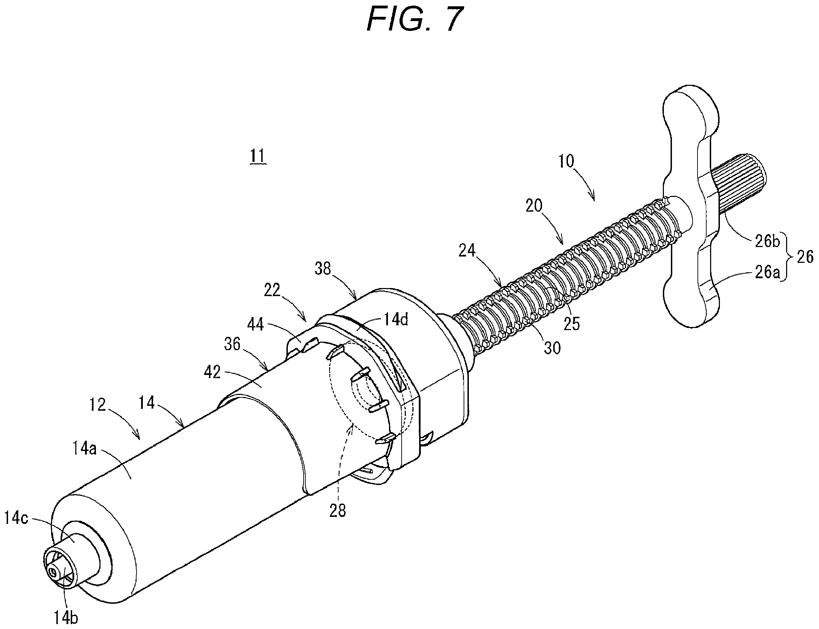

The barrel 14 includes a barrel body portion 14a having a substantially tubular shape and having a proximal end opening portion formed at a proximal end thereof, a nozzle 14b (see also FIG. 7) disposed at the distal end of the barrel body portion 14a, a lock adapter 14c disposed on the outer side of the nozzle 14b, and a flange 14d protruding radially outward from the proximal end of the barrel body portion 14a.

The barrel 14 is set to have a capacity capable of injecting a relatively large amount of drug solution M by a single injection operation. The capacity of the barrel 14 is, for example, in a range of 5 to 100 mL. Therefore, an outer diameter of the barrel body portion 14a is set to a relatively large diameter, for example, in a range of 14 to 36 mm.

The nozzle 14b is reduced in diameter from the center of the distal end of the barrel body portion 14a with respect to the barrel body portion 14a and extends in the distal end direction. The nozzle 14b is configured as a male luer which includes a circular cross-sectional outer shape and includes a tapered outer circumferential surface with an outer diameter reduced in the distal end direction. The nozzle 14b can be fitted to another device having a female luer (for example, a puncturing device 80 to be described below).

In an initial state of the syringe 12 illustrated in FIG. 1, a cap 18 is attached to the nozzle 14b, and the distal end opening portion of the nozzle 14b is liquid-tightly sealed by the cap 18. The cap 18 is detached from the nozzle 14b when the syringe 12 is used. An outer circumferential portion of the gasket 16 comes into contact with an inner circumferential surface of the barrel body portion 14a in a liquid tight manner, and the gasket 16 is disposed to be slidable in the barrel body portion 14a.

The pressurization device 10 includes a syringe plunger 20 (hereinafter, abbreviated as a "plunger 20") capable of pressing the gasket 16 in the distal end direction, and a grip 22 that can be attached to the barrel 14 and supports the plunger 20. The plunger 20 is displaced in an axial direction with respect to the grip 22 with rotation by the screwing action of the screw. The plunger 20 advances with respect to the grip 22 by a forward rotation and retracts with respect to the grip 22 by a reverse rotation.

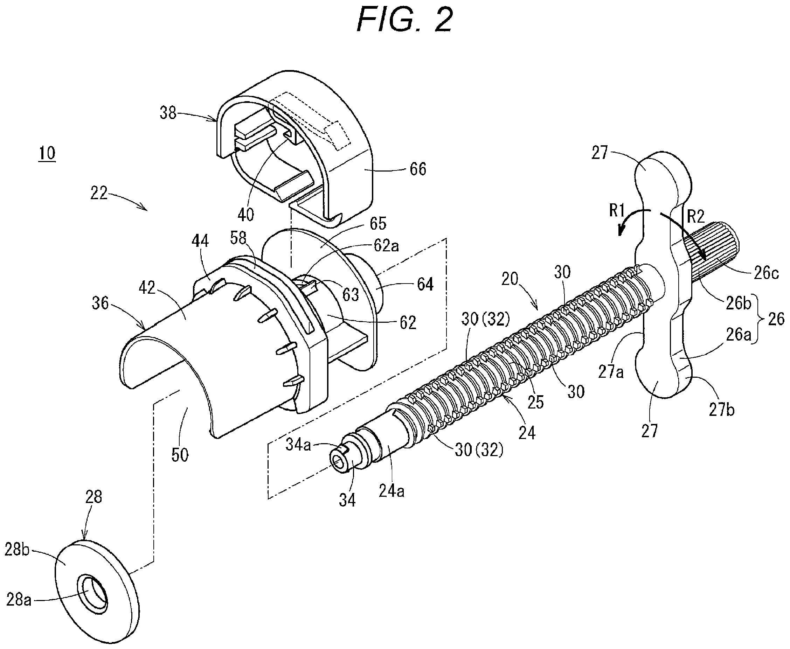

As illustrated in FIG. 2, the plunger 20 includes a plunger rod 24 having a screw thread 25 on its outer circumferential surface, a rotary operation part 26 disposed at the proximal end portion of the plunger rod 24, and a pressing part 28 disposed at the distal end portion of the plunger 20. The plunger rod 24 includes a shaft part 24a which is a rod main body, and the screw thread 25 is formed on the outer circumferential surface of the shaft part 24a. The screw thread 25 extends spirally in a range from the proximal end portion of the shaft part 24a to the vicinity of the distal end portion.

On the top of the screw thread 25, a pressing protrusion 30 protruding outward (radially outward) from the screw thread 25 is disposed. In the plunger 20, a plurality of pressing protrusions 30 is disposed. Specifically, a large number of pressing protrusions 30 is disposed at equal angle intervals (90° intervals in the illustrated example) on the screw thread 25 extending in a spiral shape. In this embodiment, a protrusion row 32 is constituted by the plurality of pressing protrusions 30 arranged on the same straight line parallel to the axis of the plunger rod 24, and a plurality of protrusion rows 32 is disposed at equal intervals in the circumferential direction.

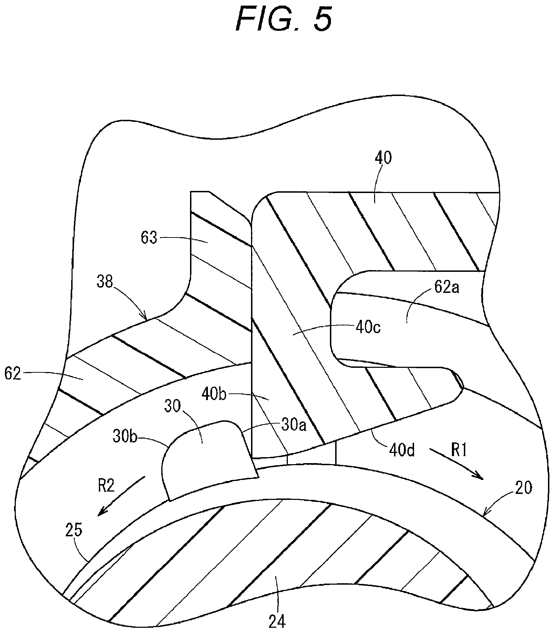

In FIG. 1, when the plunger 20 is advanced, the plunger 20 is rotated in the forward rotation direction indicated by a direction of an arrow R1, and when the plunger 20 is retracted, the plunger 20 is rotated in the reverse rotation direction indicated by a direction of an arrow R2. As illustrated in FIG. 5, an engaging part 30a substantially perpendicular to the circumferential direction of the plunger 20 is disposed on the side of the pressing protrusion 30 in the forward rotation direction (the direction of the arrow R1). On the other hand, a curved part 30b formed in an arc shape is disposed on the side of the pressing protrusion 30 in the reverse rotation direction (the direction of the arrow R2).

In FIGS. 1 and 2, the rotary operation part 26 is a part which is grabbed by a user's finger for operation in order to rotate the plunger 20. The rotary operation part 26 is disposed so as not to be rotatable with respect to the plunger rod 24. In this embodiment, the rotary operation part 26 is formed integrally with the plunger rod 24.

The rotary operation part 26 includes a finger hook rotary operation part 26a and a pinch rotary operation part 26b. The finger hook rotary operation part 26a protrudes in a direction perpendicular to the axis of the plunger rod 24. The finger hook rotary operation part 26a protrudes outward in the radial direction from the plunger rod 24 and the pinch rotary operation part 26b.

In the present embodiment, the finger hook rotary operation part 26a includes a pair of rod-like finger hook parts 27 protruding in directions opposite to each other from the plunger rod 24. A constricted part 27a having a relatively small width and a bulging part 27b having a relatively large width disposed outside the constricted part 27a are disposed at the rod-like finger hook part 27. A step difference between the constricted part 27a and the bulging part 27b makes it easier for the user to hook a finger.

The pinch rotary operation part 26b protrudes from the finger hook rotary operation part 26a in the proximal end direction coaxially with the plunger rod 24. The pinch rotary operation part 26b is formed in a substantially tubular shape. The pinch rotary operation part 26b is formed to have an appropriately small diameter so that the plunger 20 can be quickly rotated by the user's fingers in a case in which a large torque is not required when the plunger 20 is rotated. The outer diameter of the pinch rotary operation part 26b is, for example, in a range of 3 to 15 mm, preferably in a range of 7 to 10 mm. In the present embodiment, the outer diameter of the pinch rotary operation part 26b is smaller than the outer diameter of the screw thread 25.

In order to prevent easy slip of the finger when the user pinches the pinch rotary operation part 26b with the finger to rotate, a plurality of narrow grooves 26c extending along the axis of the plunger 20 is formed as an anti-slip structure on the outer circumferential surface of the pinch rotary operation part 26b at intervals in the circumferential direction. Knurling may be disposed in place of the plurality of narrow grooves 26c.

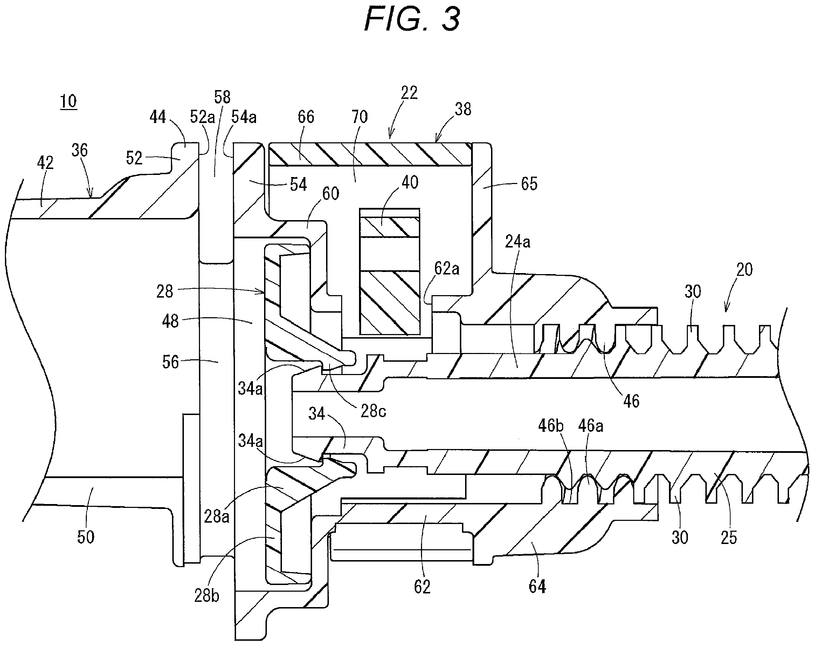

In FIG. 2, the pressing part 28 is a disk-shaped member that is rotatably supported by the distal end portion of the plunger rod 24 and has a larger diameter than that of the plunger rod 24. As illustrated in FIG. 3, a rotation support part 34 that rotatably supports the pressing part 28 is disposed at the distal end portion of the plunger rod 24 (the shaft part 24a) . A locking claw 34a protruding outward is disposed at the distal end of the rotation support part 34.

The pressing part 28 includes a tubular base part 28a constituting a center side, and a ring-shaped abutting part 28b extending outward from the tubular base part 28a. On the inner circumferential surface of the tubular base part 28a, a ring-shaped locking protrusion 28c protruding inward is disposed. The locking claw 34a and the ring-shaped locking protrusion 28c constitute a stopper means for preventing the pressing part 28 from being slipped off from the rotation support part 34 in the distal end direction.

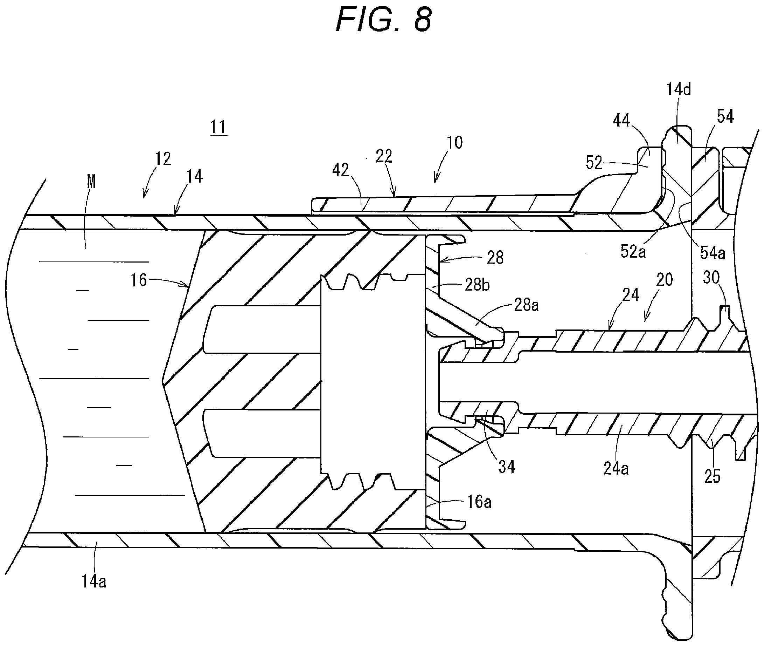

As illustrated in FIG. 8, the abutting part 28b is a portion that abuts on the proximal end surface 16a of the gasket 16 when the gasket 16 is pressed. The distal end surface of the abutting part 28b is formed in a flat shape perpendicular to the axis of the plunger 20. The outer diameter of the abutting part 28b is preferably set to be the same as the outer diameter of the proximal end surface 16a of the gasket 16, or is equal to or smaller than the outer diameter of the proximal end surface 16a of the gasket 16 and is close to the outer diameter. In the present embodiment, the outer diameter of the abutting part 28b is set to be approximately the same as the outer diameter of the proximal end surface 16a of the gasket 16.

In FIG. 1, the grip 22 is configured to be attachable to the barrel 14 from a direction perpendicular to the axis of the barrel 14. As illustrated in FIG. 2, the grip 22 includes a first member 36 constituting a grip main body portion, and a second member 38 fixed to the first member 36 and provided with a displacement mechanism part 40.

As illustrated in FIG. 3, the first member 36 includes a barrel attachment part 42 attachable to the outer circumferential surface of the barrel 14, a flange attachment part 44 attachable to the flange 14d, a female screwing part 46 screwed to the screw thread 25 of the plunger 20, and an accommodation part 48 formed between the flange attachment part 44 and the female screwing part 46.

The barrel attachment part 42 and the flange attachment part 44 are formed with attachment openings 50 (see also FIG. 2) in which the barrel attachment part 42 and the flange attachment part 44 are cut in the axial direction. The barrel attachment part 42 extends in the axial direction by a predetermined length, is curved along the outer circumferential surface of the barrel 14, and is formed in a C-shaped cross section.

The flange attachment part 44 includes a distal end side holding part 52, a proximal end side holding part 54, and a connecting part 56 which connects the distal end side holding part 52 and the proximal end side holding part 54. A holding groove 58 is formed between the distal end side holding part 52 and the proximal end side holding part 54. As illustrated in FIG. 8, in a state in which the grip 22 is attached to the barrel 14, the flange 14d is inserted into the holding groove 58, and the flange 14d is sandwiched and held between the holding surfaces 52a and 54a facing each other. As a result, a relative movement of the grip 22 in the axial direction with respect to the barrel 14 is restricted.

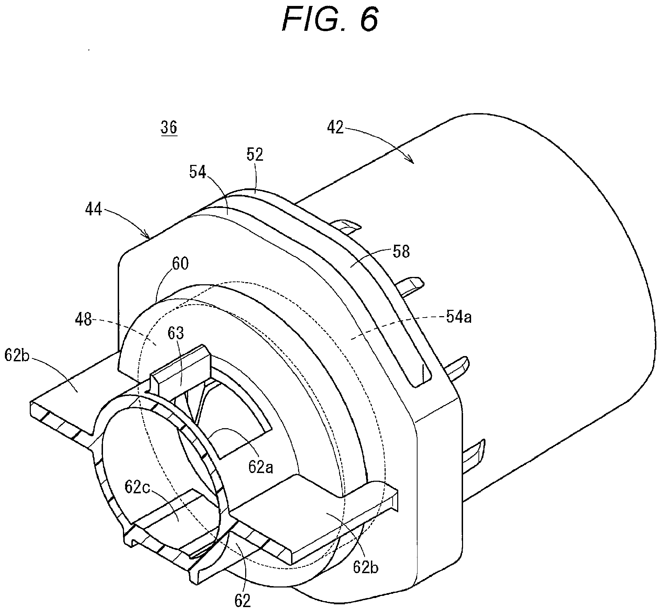

As illustrated in FIG. 3, in the first member 36, an accommodation part forming part 60 is disposed to be adjacent to the proximal end side of the flange attachment part 44, a first tubular part 62 (see also FIG. 2) is disposed to be adjacent to the proximal end side of the accommodation part forming part 60, and a second tubular part 64 (see also FIG. 2) is disposed to be adjacent to the proximal end side of the first tubular part 62. The accommodation part 48 capable of accommodating the aforementioned pressing part 28 of the plunger 20 is formed inside the accommodation part forming part 60. As illustrated in FIG. 6, the accommodation part 48 is a circular groove which is recessed in the proximal end direction with respect to the flange attachment part 44 (specifically, the holding surface 54a of the proximal end side holding part 54) and has an inner diameter larger than the outer diameter of the pressing part 28.

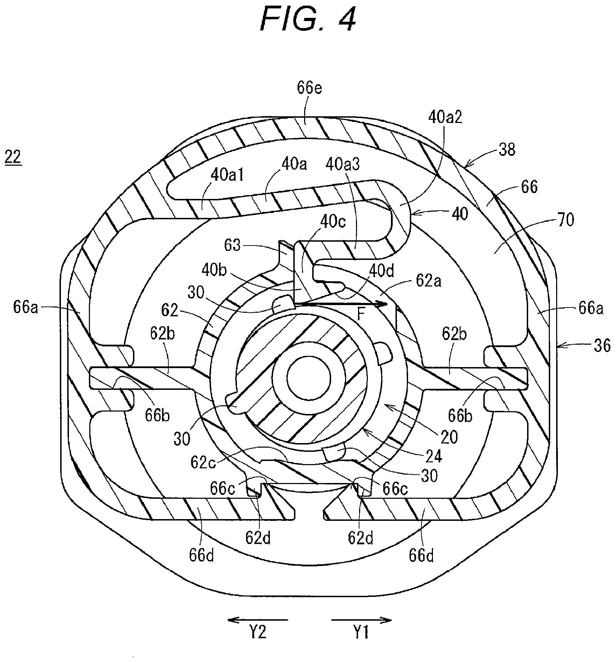

As illustrated in FIGS. 4 and 6, a hole part 62a penetrating a circumferential wall of the first tubular part 62 in the thickness direction is disposed in the first tubular part 62. The displacement mechanism part 40 of the second member 38 is inserted into the hole part 62a. A wall 63 is disposed in the first tubular part 62 to be adjacent to the hole part 62a. The wall 63 protrudes outward from the outer circumferential surface of the first tubular part 62. A pair of fixing ribs 62b protrudes from the outer circumferential surface of the first tubular part 62 in the directions opposite to each other. The wall 63 is disposed at a substantially central position between the pair of fixing ribs 62b in the circumferential direction of the first tubular part 62.

As illustrated in FIG. 4, a rod position restricting part 62c which restricts the position of the plunger rod 24 with respect to the pressed part 40b is disposed on the side opposite to the pressed part 40b on the basis of the plunger rod 24. The rod position restricting part 62c is a portion padded on an arcuate inner circumferential surface of the first tubular part 62. When coming into contact with the pressing protrusion 30, the rod position restricting part 62c restricts the position of the plunger rod 24 so that the pressing protrusion 30 on the opposite side thereof can press the displacement mechanism part 40.

As illustrated in FIG. 3, a female screwing part 46 is formed on the inner circumferential surface of the second tubular part 64. The female screwing part 46 includes a screw thread 46a extending in a spiral shape. A groove 46b between the screw threads 46a adjacent to each other in the axial direction has a depth that permits the pressing protrusion 30 of the plunger 20 to enter. As the female screwing part 46, instead of the screw thread 46a extending in a spiral shape, a protruding part to be screwed to the screw thread 25 as the male screwing part of the plunger 20 may be disposed.

As illustrated in FIGS. 2 and 4, the second member 38 includes a fixing base part 66 which is fixed to the first member 36 and supports the displacement mechanism part 40. The displacement mechanism part 40 extends from the inner surface of the fixing base part 66. The fixing base part 66 is formed in a C shape to partially surround the first tubular part 62 of the first member 36.

As illustrated in FIG. 4, a pair of fitting grooves 66b is disposed on the inner surfaces of the mutually facing side walls 66a of the fixing base part 66. A pair of fixing ribs 62b of the first member 36 is fitted to the pair of fitting grooves 66b, respectively. Therefore, the second member 38 is positioned with respect to the first member 36 in a direction perpendicular to the axis of the first member 36.

A pair of engaging claws 66c is disposed at both circumferential end portions of the fixing base part 66. Specifically, the pair of engaging claws 66c is formed at the end portions of a pair of arms 66d which is curved from the side walls 66a on both sides and extends inward. The pair of engaging claws 66c is engaged with an engaging protrusion 62d disposed on the first member 36 (specifically, the first tubular part 62). Thus, the fixing of the second member 38 to the first member 36 becomes more rigid. Further, the engaging claw 66c and the engaging protrusion 62d may not be disposed.

As illustrated in FIG. 3, the second member 38 is held between a proximal end side holding part 54 and a flange-like wall part 65 protruding outward from the outer circumferential surface of the second tubular part 64. As a result, the second member 38 is positioned with respect to the first member 36 in the axial direction of the first member 36. An internal space 70 surrounded by the first member 36 and the second member 38 is formed inside the second member 38, and the displacement mechanism part 40 is disposed in the internal space 70.

As illustrated in FIG. 4, the displacement mechanism part 40 extends from a dome-shaped intermediate wall part 66e that connects the side walls 66a on both sides of the fixing base part 66. The displacement mechanism part 40 is displaced substantially along a direction of a tangential force F acting on the pressing protrusion 30 which starts to press the displacement mechanism part 40 at the time of the forward rotation of the plunger rod 24. In the present embodiment, the displacement mechanism part 40 includes an elastically deformable elastic piece part 40a functioning as a spring part, and a pressed part 40b and an abutting part 40c disposed on the free end side of the elastic piece part 40a.

The elastic piece part 40a includes a U-shaped curved part which opens in the direction substantially opposite to the direction of the tangential force F acting on the pressing protrusion 30 which starts to press the displacement mechanism part 40 at the time of the forward rotation of the plunger rod 24. The elastic piece part 40a extends from the support part (the fixing base part 66) along the direction (a direction of an arrow Y1) in which the pressed part 40b is pressed and displaced at the time of the forward rotation of the plunger 20. The fixed end portion of the elastic piece part 40a is disposed at a position away from the wall 63 in a direction (a direction of an arrow Y2) opposite to the direction in which the pressed part 40b is pressed and displaced at the time of the forward rotation of the plunger 20. The elastic piece part 40a is folded back at a position away from the wall 63 in a direction (the direction of the arrow Y1) in which the pressed part 40b is pressed and displaced at the time of the forward rotation of the plunger 20.

Specifically, the elastic piece part 40a includes a first extending part 40a1 which extends from the support part (the fixing base part 66) along the direction in which the pressed part 40b is pressed and displaced at the time of the forward rotation of the plunger rod 24, a curved extending part 40a2 which extends from an extending distal end of the first extending part 40a1 while being curved in a direction opposite to the extending direction of the first extending part 40a1, and a second extending part 40a3 which extends from the extending distal end of the curved extending part 40a2 in a direction opposite to the extending direction of the first extending part 40a1. The pressed part 40b is disposed at the extending distal end of the second extending part 40a3.

The pressed part 40b is a portion that is pressed by the pressing protrusion 30 when the plunger rod 24 rotates. A tapered part 40d is disposed to be adjacent to the pressed part 40b. The tapered part 40d is inclined so as to be separated from the screw thread 25 in a direction (the direction of the arrow Y1) in which the pressed part 40b is pressed and displaced at the time of the forward rotation of the plunger 20.

The abutting part 28b is a portion that abuts on the wall 63 when the displacement mechanism part 40 repelled by the pressing protrusion 30 collides against the wall 63. In the state illustrated in FIG. 4 in which the pressed part 40b is not pressed by the pressing protrusion 30, the abutting part 28b comes into contact with the wall 63 and the pressed part 40b is positioned inside the first tubular part 62.

The constituent material of the first member 36 is not particularly limited, but examples thereof include resin materials such as polypropylene, polycarbonate, polyamide, polysulfone, polyarylate, ABS, and high-density polyethylene.

The constituent materials of the second member 38 are not particularly limited, but may be selected from the above-mentioned materials exemplified as the constituent material of the first member 36. The constituent material of the second member 38 is preferably a material that is softer and is easily elastically deformed (for example, polyacetal or the like) than the constituent material of the first member 36. By configuring the first member 36 and the second member 38 with different materials, it is possible to have suitable mechanical characteristics for each.

Next, the operation of the pressurization device 10 configured as described above will be described. In order to use the syringe 12, the user attaches the pressurization device 10 in which the grip 22 and the plunger 20 are assembled as illustrated in FIG. 7 to the syringe 12. Specifically, the pressurization device 10 is attached to the proximal end portion of the barrel 14 (the proximal end outer circumferential surface of the barrel 14 and the flange 14d) from a direction perpendicular to the axis of the barrel 14.

In this case, in the pressurization device 10 according to the present embodiment, the accommodation part 48 (see FIG. 3) capable of accommodating the pressing part 28 of the plunger 20 is disposed between the flange attachment part 44 and the female screwing part 46 of the grip 22. Thus, as illustrated in FIG. 3, the pressing part 28 of the plunger 20 is in a state of being accommodated in the accommodation part 48 of the grip 22 (a state in which the distal end surface of the pressing part 28 is located at the proximal end side of the holding surface 54a of the proximal end side holding part 54). As a result, as illustrated in FIG. 7, the pressurization device 10 in which the grip 22 and the plunger 20 are assembled can be attached to the barrel 14 from a direction perpendicular to the axis of the barrel 14 without any trouble. Therefore, one-touch attachment of the pressurization device 10 to the syringe 12 is possible. Further, since the pressurization device 10 is attached to the barrel 14 from the direction perpendicular to the axis of the barrel 14, the pressurization device 10 is not unintentionally detached from the barrel 14 during use.

On the other hand, in the screw threaded syringe of JP 2005-535415 A, a male screw is disposed on the outer circumferential surface of the shaft of the plunger, and a gasket (a liquid-tight component 56) is connected to the distal end portion of the plunger. In the screw threaded syringe of JP 2005-535415 A, when assembling each component to use a threaded plunger, it is necessary to insert the plunger into the grip after attaching the grip to the syringe barrel. Therefore, unlike the pressurization device 10, in the screw threaded syringe of JP 2005-535415 A, it is not possible to attach the plunger to the grip with one touch after assembling the plunger and the grip.

In this embodiment, in a state in which the pressing part 28 is accommodated deepest with respect to the accommodation part 48, the distal end surface of the pressing part 28 is located at the proximal end side of the most distal end position of the accommodation part 48 (see FIG. 3). Therefore, when the pressurization device 10 is attached to the barrel 14, it is possible to reliably prevent the pressing part 28 from being caught by the proximal end portion of the barrel 14.

Next, the user detaches the cap 18 (see FIG. 1) from the distal end portion (the nozzle 14b) of the syringe 12, connects the distal end portion of the syringe 12 to another device (for example, a puncturing device 80 to be described later), and performs a necessary preparatory work (for example, priming to be described later). Further, as illustrated in FIG. 8, by rotating the plunger 20 in the forward direction, the plunger 20 is advanced and the gasket 16 is pressed in the barrel 14 in the distal end direction. As a result, the gasket 16 advances inside the barrel 14, and the interior of the barrel 14 is pressurized, and thus, the drug solution M is discharged from the nozzle 14b.

Meanwhile, in the screw threaded syringe of JP 2005-535415 A, in order to tactilely or audibly provide a doctor with the rotating condition of the plunger, a mechanism for generating a click sound when the plunger rotates is disposed. In the mechanism, a click sound is generated by an impact at the time when the click piece is engaged with the groove disposed in the plunger. However, according to the mechanism disclosed in JP 2005-535415 A, the magnitude of the generated click sound differs depending on the rotational speed of the plunger, and when the plunger is rotated slowly, almost no click sound is generated.

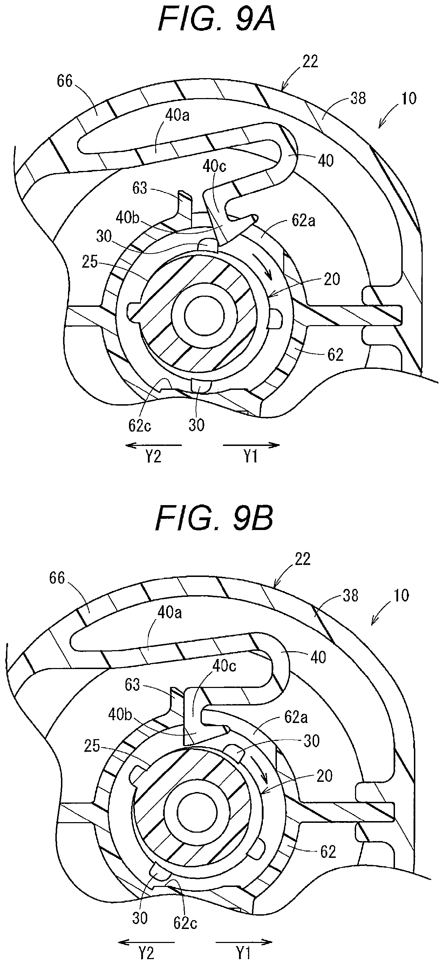

In contrast, according to the pressurization device 10 of the present embodiment, when the plunger 20 is rotated in the forward direction for advancement, the pressurization device 10 generates a click sound (and click feeling). That is, when the plunger 20 is rotated for advancement of the gasket 16 inside the syringe 12, as illustrated in FIGS. 9A and 9B, the displacement mechanism part 40 disposed in the grip 22 is repelled by the pressing protrusion 30 disposed on the screw thread 25 of the plunger 20. Further, the displacement mechanism part 40 repelled by the pressing protrusion 30 is displaced by the elastic restoring force and collides against the wall 63 disposed on the grip 22. Therefore, irrespective of the rotational speed of the plunger 20, the magnitude of the generated click sound is constant, and even when the plunger 20 is slowly rotated, the click sound can be reliably generated.

The generation of the click sound will be described in more detail. As illustrated in FIG. 9A, when the plunger 20 rotates in the forward direction, the pressing protrusion 30 disposed on the screw thread 25 presses the pressed part 40b of the displacement mechanism part 40, while moving in the circumferential direction. As a result, in the displacement mechanism part 40, the pressed part 40b is pressed by the pressing protrusion 30 and displaced in the direction (the direction of the arrow Y1) away from the wall 63 with elastic deformation of the elastic piece part 40a. The displacement of the pressed part 40b is performed while the engagement (contact) between the pressing protrusion 30 and the pressed part 40b is maintained.

Further, when the displacement of the pressing protrusion 30 progresses and the engagement between the pressing protrusion 30 and the pressed part 40b is released, as illustrated in FIG. 9B, by the elastic restoring force of the elastic piece part 40a, the displacement mechanism part 40 (the abutting part 28b) is displaced in the direction (the direction of arrow Y2) opposite to the direction of pressing by the pressing protrusion 30 and collides against the wall 63. The sound generated with the collision is provided to the user as a click sound. The click sound is generated each time the plunger 20 is rotated by a predetermined angle (90° in the present embodiment) in the forward rotation direction.

Therefore, even if the user does not see the scale disposed in the syringe 12, the user can adjust the amount of discharge of the drug solution M, using the heard click sound as an index. In addition, because the amount of discharge can be grasped by sound (and feeling), the amount of discharge can be adjusted more accurately than the case of adjusting the amount of discharge by observing the scale. For example, the syringe 12 is designed to discharge 1 mL of the drug solution M when the plunger 20 makes one revolution, and the pressing protrusion 30 is disposed to generate a click sound once each time the plunger 20 is rotated by 90°. In this case, in order to additionally discharge 0.5 mL from a certain point of time, the user may rotate the plunger 20 so that a click sound is generated twice.

In the present embodiment, the displacement mechanism part 40 is displaced substantially along the direction of the tangential force F (see FIG. 4) acting on the pressing protrusion 30 which starts to press the displacement mechanism part 40 at the time of the forward rotation of the plunger rod 24. With this configuration, since the force received by the pressing protrusion 30 from the displacement mechanism part 40 increases, and the amount of displacement of the displacement mechanism part 40 also increases, a larger click sound can be generated.

In the present embodiment, the displacement mechanism part 40 includes an elastic piece part 40a extending from the support part (the fixing base part 66). Therefore, elastic energy can be accumulated with a simple configuration when pressed by the pressing protrusion 30. Moreover, since the elastic piece part 40a includes a U-shaped curved part, it is easy to balance well the easiness of deformation of the displacement mechanism part 40 and the strength of the elastic restoring force. Thus, it is possible to generate a clear click sound, while suppressing the increase in the rotation resistance of the plunger 20.

In the present embodiment, a rod position restricting part 62c which restricts the position of the plunger rod 24 with respect to the pressed part 40b is disposed on the side opposite to the pressed part 40b on the basis of the plunger rod 24. Therefore, when the plunger rod 24 rotates, it is possible to reliably press the pressed part 40b in the circumferential direction by the pressing protrusion 30.

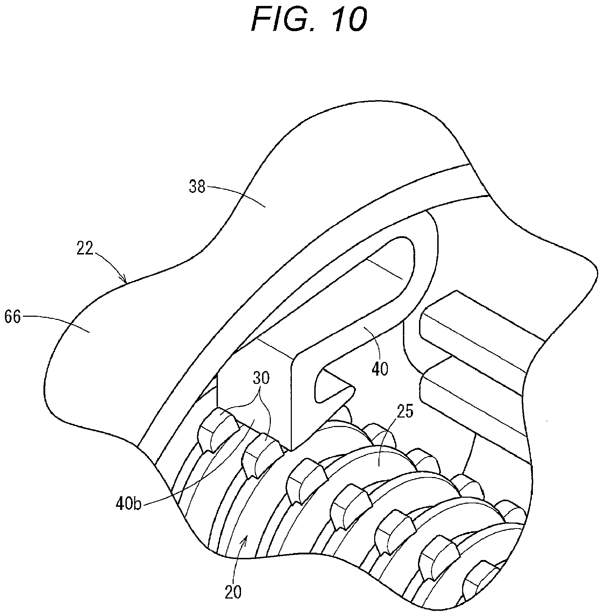

In the present embodiment, as illustrated in FIG. 10, when the plunger 20 rotates in the forward direction, the plurality of pressing protrusions 30 presses the displacement mechanism part 40. Therefore, at the time of forward rotation of the plunger 20, the pressing protrusion 30 can be made to reliably abut on the displacement mechanism part 40. In addition, when the pressed part 40b is pressed, the load applied to each pressing protrusion 30 decreases, and breakage of the pressing protrusion 30 can be prevented.

In the present embodiment, the grip 22 includes an internal space 70 (see FIG. 4) in which the displacement mechanism part 40 is disposed to echo the sound generated with collision of the displacement mechanism part 40 against the wall 63. Therefore, it is possible to amplify a sound pressure level of the click sound generated by the collision of the displacement mechanism part 40 against the wall 63 in the internal space 70, and to provide the user with a clear click sound.

In the present embodiment, the plunger 20 and the pressing part 28 are rotatable relative to each other, and the frictional resistance between the gasket 16 and the pressing part 28 is larger than the frictional resistance between the pressing part 28 and the plunger rod 24. Therefore, as illustrated in FIG. 8, when the gasket 16 is pressed with the rotation of the plunger 20, the gasket 16 advances without rotating inside the barrel 14. Therefore, deformation of the gasket 16 can be suppressed, and the liquid-tightness between the inner surface of the barrel 14 and the gasket 16 can be favorably secured.

In the present embodiment, by rotating the plunger 20 in the reverse direction at the point of time when a predetermined amount of the drug solution M is discharged, the pressure inside the barrel 14 can be immediately released, and the discharge of the drug solution M can be quickly stopped. That is, when the plunger 20 is rotated in the reverse direction from the state in which the plunger 20 is rotated in the forward direction to discharge the drug solution M in the barrel 14, the plunger 20 retracts with respect to the barrel 14. At this time, since the gasket 16 retracts by the pressure of the drug solution M increased at the time of the forward rotation of the plunger 20, the pressurization in the barrel 14 is released and the discharge of the drug solution M is stopped. Therefore, it is possible to release the pressurization by a simple operation and to prevent unintentional release of pressurization. In addition, since the gasket 16 is retracted by the pressure inside the barrel 14 without pulling the gasket 16 in the proximal end direction with the plunger 20, it is possible to prevent suction of the drug solution M due to excessive retraction of the plunger 20.

The drug solution M has a viscosity in a range of 50 to 120 mPa·s. Since the drug solution M has such a viscosity, the pressure of the drug solution M increased at the time of the forward rotation of the plunger 20 is maintained until immediately before the reverse rotation. Therefore, at the time of reverse rotation, the gasket 16 is reliably retracted in the proximal end direction by the pressure of the drug solution M, and the pressurization is released.

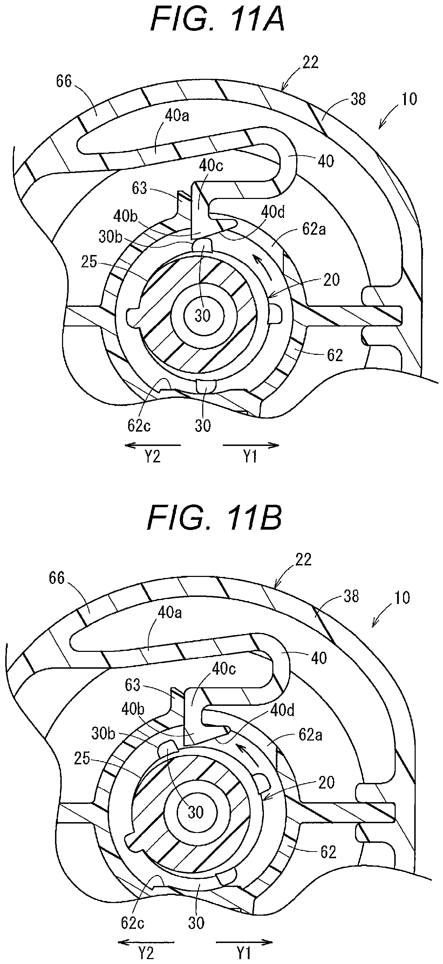

In the present embodiment, when the plunger 20 is rotated in the reverse direction, as illustrated in FIGS. 11A and 11B, the displacement mechanism part 40 is displaced radially outward of the plunger 20 and gets on the pressing protrusion 30. That is, since the reverse rotation of the plunger 20 is not inhibited by the pressing protrusion 30 (the plunger 20 idles in relation to the pressing protrusion 30), the plunger 20 is rotated in the reverse direction to release the pressure inside the syringe 12, and the discharge of the drug solution M can be stopped. Further, when the plunger 20 rotates in the reverse direction, since the displacement mechanism part 40 does not collide against the wall 63, the plunger 20 can idle without generating a clear sound like a click sound.

In this case, as illustrated in FIG. 11A, first, the displacement mechanism part 40 gets on the pressing protrusion 30, while being elastically deformed. Since the curved part 30b is disposed at the pressing protrusion 30 and the tapered part 40d is disposed at the displacement mechanism part 40, the pressing protrusion 30 smoothly gets on. Further, as illustrated in FIG. 11B, when the pressing protrusion 30 further displaces in the circumferential direction with the reverse rotation of the plunger 20, the displacement mechanism part 40 falls (comes off) from the pressing protrusion 30, and returns to the original shape (displaces to the plunger 20 side) by the elastic restoring force. At this time, even if the displacement mechanism part 40 comes into contact with the screw thread 25, the impact caused by the contact is considerably smaller than the impact at the time when the abutting part 28b collides against the wall 63 at the time of the forward rotation of the plunger 20.

In the present embodiment, the elastic piece part 40a extends from the support part (the fixing base part 66) along the direction in which the pressed part 40b is pressed and displaced at the time of the forward rotation of the plunger rod 24. Therefore, when the plunger 20 rotates in the reverse direction, the displacement mechanism part 40 is easily deformed radially outward of the plunger 20 along the wall 63. Therefore, the plunger 20 can be rotated in the reverse direction without applying a large torque.

In the present embodiment, as illustrated in FIG. 1, the rotary operation part 26 includes a finger hook rotary operation part 26a protruding in a direction perpendicular to the axis of the plunger rod 24, and a pinch rotary operation part 26b protruding in the proximal end direction from the finger hook rotary operation part 26a coaxially with the plunger rod 24. The screw thread 25 can be screwed with a female screwing part 46 disposed in the syringe 12 or attached to the syringe 12, and the plunger 20 can be advanced in the distal end direction by the screwing action between the screw thread 25 and the female screwing part 46 to press the gasket 16 in the distal end direction. Further, the outer diameter of the pinch rotary operation part 26b is smaller than the outer diameter of the finger hook rotary operation part 26a. Therefore, when it is not necessary to apply a large torque to the plunger 20, by operating the pinch rotary operation part 26b, the plunger 20 can be rotated quickly and the plunger 20 can be advanced quickly. Therefore, the treatment using the syringe 12 can be efficiently performed. That is, depending on the situations, it is possible to select to apply a large torque to the syringe plunger by operating the finger hook rotary operation part 26a and to quickly rotate and advance the syringe plunger by operating the pinch rotary operation part 26b.

In the present embodiment, the finger hook rotary operation part 26a protrudes rather than the screw thread 25 in a direction perpendicular to the axis of the plunger rod 24. Therefore, a larger torque can be applied to the plunger 20.

In particular, in the present embodiment, the outer diameter of the pinch rotary operation part 26b is smaller than the outer diameter of the screw thread 25. Further, the pinch rotary operation part 26b includes a cylindrical shape. Therefore, when the pinch rotary operation part 26b is operated by being pinched, the plunger 20 can be easily and more quickly rotated. In addition, since an anti-slip structure for the finger is disposed at the outer circumferential portion of the pinch rotary operation part 26b, when the pinch rotary operation part 26b is operated by being pinched, slippage of the finger is suppressed and the operation is easily performed.

Further, the finger hook rotary operation part 26a includes a pair of rod-like finger hook parts 27 protruding in opposite directions to each other from the plunger rod 24. For this reason, it is easy to apply a large torque to the plunger 20 when operating the finger hook rotary operation part 26a by hooking a finger. In particular, since a constricted part 27a and a bulging part 27b disposed outside the constricted part 27a are disposed in the pair of rod-like finger hook parts 27, when operating the finger hook rotary operation part 26a by hooking a finger, the user can easily hook his/her finger.

Next, as an example of a treatment method using the syringe 12 and the pressurization device 10, a case in which an endoscopic submucosal dissection (ESD) or an endoscopic mucosal resection (EMR) is performed will be described. First, the endoscopic submucosal dissection will be described.

The user (surgeon or assistant) attaches the pressurization device 10 to the syringe 12 (pre-filled syringe) filled with the sodium hyaluronate solution M1 and detaches the cap 18 from the syringe 12.

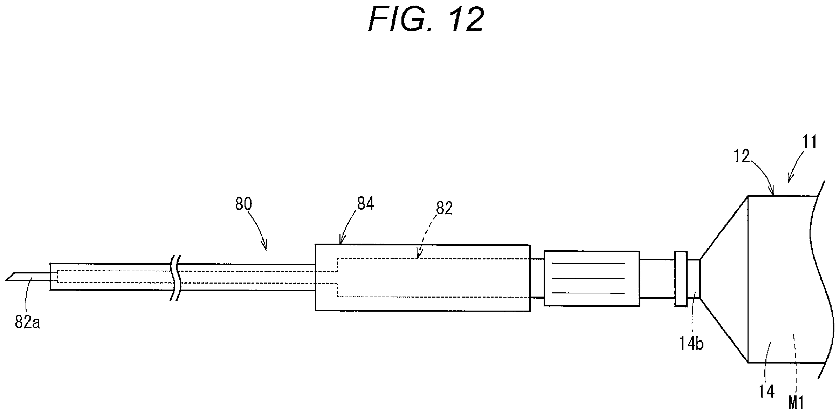

Next, as illustrated in FIG. 12, the user connects the distal end portion (the nozzle 14b) of the syringe 12 to the proximal end portion of the inner catheter 82 of the puncturing device 80, and in the state of causing the needle 82a to protrude from the outer catheter 84, the plunger 20 is advanced by being rotated, and priming (filling the lumen of the inner catheter 82 and the needle 82a with the sodium hyaluronate solution M1) is performed. In this case, until the sodium hyaluronate solution M1 reaches the needle 82a, since the rotary operation of the plunger 20 requires only a comparatively small operation force, the user may rotate the plunger 20 by picking the pinch rotary operation part 26b illustrated in FIG. 1 or the like with a finger. As a result, the plunger 20 can be quickly rotated and the plunger 20 can be advanced quickly.

Further, as illustrated in FIG. 12, the puncturing device 80 includes an inner catheter 82 and an outer catheter 84. The inner catheter 82 is axially slidable with respect to the outer catheter 84 within a restricted range. The needle 82a is connected to the distal end of the inner catheter 82. When the inner catheter 82 is located at the retracted position with respect to the outer catheter 84, the needle 82a is accommodated within the outer catheter 84. When the inner catheter 82 is located at the advanced position with respect to the outer catheter 84, the needle 82a protrudes from the distal end of the outer catheter 84.

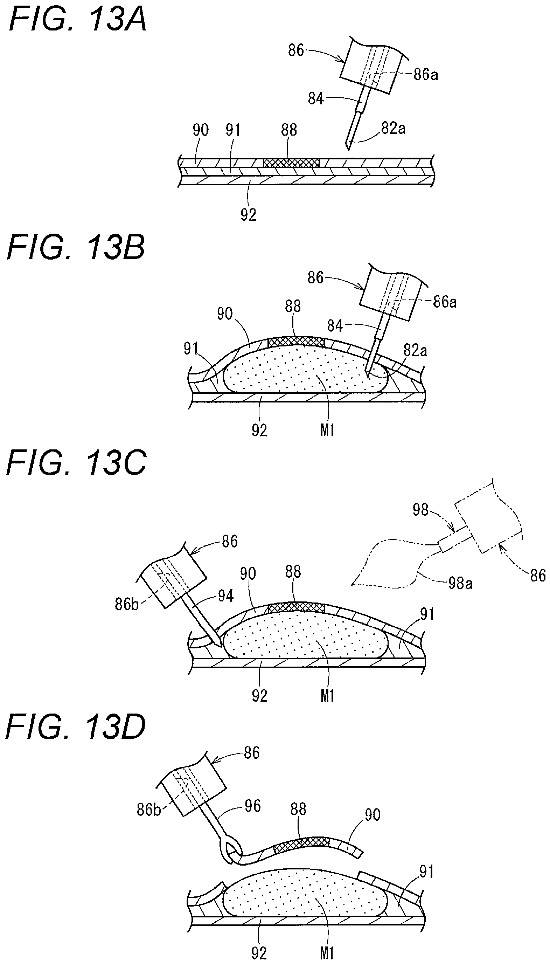

Next, the user retracts the inner catheter 82 to accommodate the needle 82a in the outer catheter 84. Further, the user inserts the puncturing device 80 into the insertion hole 86a (see FIG. 13A) of the endoscope 86.

Next, the user who is a surgeon causes the needle 82a to protrude from the distal end of the endoscope 86 in the digestive tract of the patient as illustrated in FIG. 13A. Further, the user approaches the mucous membrane 90 around the lesioned part 88, and advances the needle 82a toward the mucous membrane 90. As a result, the needle 82a penetrates the mucous membrane 90 and is punctured into a submucosal layer 91.

In this state, as illustrated in FIG. 13B, a predetermined amount of sodium hyaluronate solution M1 is injected into the submucosal layer 91 to raise the lesioned part 88. Specifically, when the hyaluronate solution M1 in the syringe 12 is pressurized by the pressurization device 10 (the plunger 20 is rotated in the forward direction to advance the gasket 16), the sodium hyaluronate solution M1 is injected into the submucosal layer 91 from the distal end opening of the needle 82a. The lesioned part 88 is lifted by injection of sodium hyaluronate solution M1. That is, the submucosal layer 91 under the lesioned part 88 is peeled off from the muscular layer 92 and the lesioned part 88 is raised.

In this case, the syringe 12 has a moderate length (a length not too long) which is easy to handle, and the syringe 12 is filled with the sodium hyaluronate solution M1 in an amount sufficient to raise the lesioned part 88 by a single injection. Therefore, the inner diameter of the barrel 14 also has a size corresponding to such an amount. Therefore, in order to inject the sodium hyaluronate solution M1 having a relatively high viscosity via the needle 82a, it is necessary to press the gasket 16 in the distal end direction with a large force. In the present embodiment, since the pressurization device 10 is used, it is possible to advance the gasket 16 and inject the sodium hyaluronate solution M1 via the needle 82a without requiring an excessive operating force in the operation of the plunger 20.

At the point of time when a predetermined amount of sodium hyaluronate solution M1 is injected, the user rotates the plunger 20 in the reverse direction to release the pressurization. As a result, the discharge of the sodium hyaluronate solution M1 from the syringe 12 stops immediately, and the injection amount of the sodium hyaluronate solution M1 into the submucosal layer 91 can be accurately controlled.

Next, as illustrated in FIG. 13C, the user inserts a peeling device 94 (an acicular scalpel or the like) into a forceps hole 86b of the endoscope 86, and cuts the mucous membrane 90 to surround the lesioned part 88, using the peeling device 94. Next, as illustrated in FIG. 13D, the user inserts a gripping device 96 into the forceps hole 86b of the endoscope 86, peels off the mucous membrane 90 having the lesioned part 88, using the gripping device 96, and extracts the peeled mucous membrane 90 to the outside of the body.

On the other hand, when the endoscopic mucosal resection is performed, an ablation device 98 indicated by an imaginary line in FIG. 13C is used instead of the peeling of the mucous membrane 90 by the peeling device 94 described above. The ablation device 98 includes a snare ring 98a (a loop-shaped electric scalpel). The user causes the snare ring 98a to protrude from the endoscope 86 in the patient's body and hooks the snare ring 98a to the mucous membrane 90 around the raised lesioned part 88. Further, when the snare ring 98a is squeezed and a high-frequency current flows through the snare ring 98a, the mucous membrane 90 having the lesioned part 88 is cut. The cut mucous membrane 90 is collected by the gripping device 96 illustrated in FIG. 13D. Other procedure processes are performed in the same manner as the above-described endoscopic submucosal dissection.

The present invention is not limited to the above-described embodiments, and various modifications can be made within the scope of the present invention defined by the appended claims.

### Description of the Reference Symbols

- 10:: Syringe pressurization device
- 12:: Syringe
- 14:: Barrel
- 14d:: Flange
- 16:: Gasket
- 20:: Syringe plunger
- 22:: Grip
- 24:: Plunger rod
- 25:: Screw thread
- 26:: Rotary operation part
- 26a:: Finger hook rotary operation part
- 26b:: Pinch rotary operation part
- 28:: Pressing part
- 30:: Pressing protrusion
- 40:: Displacement mechanism part
- 63:: Wall
- M:: Drug solution

## Claims

1. A syringe pressurization device (10) configured to be attached to a syringe (12) including a barrel (14) and a gasket (16) sliding inside the barrel (14), the syringe pressurization device (10) comprising:
a plunger (20) configured to advance the gasket (16) in a distal end direction; and
a grip (22) attachable to the barrel (14) from a direction perpendicular to an axis of the barrel (14) and supporting the plunger (20),
wherein the plunger (20) includes a plunger rod (24) including a screw thread (25) disposed on an outer circumferential surface thereof, and a pressing part (28) disposed at a distal end portion of the plunger rod (24) and configured to press the gasket (16) in a distal end direction,
wherein the plunger (20) is a syringe plunger (20) comprising:
a rotary operation part (26) disposed at a proximal end portion of the plunger rod (24),
wherein the rotary operation part (26) includes a finger hook rotary operation part (26a) protruding in a direction perpendicular to an axis of the plunger rod (24), and a pinch rotary operation part (26b) protruding in a proximal end direction from the finger hook rotary operation part (26a) coaxially with the plunger rod (24),
the screw thread (25) is configured to be screwed with a female screwing part (46),
the syringe plunger (20) is configured to advance in a distal end direction by a screwing action between the screw thread (25) and the female screwing part (46) so as to press the gasket (16) in a distal end direction, and
an outer diameter of the pinch rotary operation part (26b) is smaller than an outer diameter of the finger hook rotary operation part (26a), and wherein
said grip (22) includes:
a flange attachment part (44) attachable to a flange disposed at a proximal end portion of the barrel (14),
a female screwing part (46) disposed proximal of the flange attachment part (44) and screwed to the screw thread (25), and
an accommodation part (48) disposed between the flange attachment part (44) and the female screwing part (46) and configured to accommodate the pressing part (28).

2. The syringe pressurization device (10) according to claim 1 wherein in said syringe plunger (20) the finger hook rotary operation part (26a) protrudes rather than the screw thread (25) in a direction perpendicular to the axis of the plunger rod (24).

3. The syringe pressurization device (10) according to claim 1 or 2, wherein in said syringe plunger (20) the outer diameter of the pinch rotary operation part (26b) is smaller than the outer diameter of the screw thread (25).

4. The syringe pressurization device (10) according to any one of claims 1 to 3, wherein in said syringe plunger (20) the pinch rotary operation part (26b) has a cylindrical shape.

5. The syringe pressurization device (10) according to claim 4, wherein in said syringe plunger (20) an anti-slip structure for a finger is disposed on an outer circumferential portion of the pinch rotary operation part (26b).

6. The syringe pressurization device (10) according to any one of claims 1 to 5, wherein in said syringe plunger (20) the finger hook rotary operation part (26a) includes a pair of rod-like finger hook parts (27) protruding in directions opposite to each other from the plunger rod (24).

7. The syringe pressurization device (10) according to claim 6, wherein in said syringe plunger (20) a constricted part (27a), and a bulging part (27b) disposed outside the constricted part (27a) are disposed in the pair of rod-like finger hook parts (27).

8. The syringe pressurization device (10) according to any of the preceding claims, wherein the plunger rod (24) includes a rotation support part (34) at a distal end portion thereof,
the pressing part (28) is a disc-shaped member supported on the rotation support part (34) of the plunger rod (24) to be rotatable about the axis of the plunger rod (24) and having a larger diameter than that of the plunger rod (24), and
the accommodation part (48) is a circular groove recessed in the proximal end direction with respect to the flange attachment part (44) and having an inner diameter larger than an outer diameter of the pressing part (28).

9. The syringe pressurization device (10) according to claim 8, wherein the rotation support part (34) includes a locking claw (34a) protruding outward at a distal end thereof,
the pressing part (28) includes a tubular base part (28a) comprising a center side thereof, and a ring-shaped abutting part (28b) extending outward from the tubular base part (28a) and configured to abut on the proximal end surface of the gasket (16),
a ring-shaped locking protrusion (28c) protruding inward is disposed on an inner circumferential surface of the tubular base part (28a), and
the pressing part (28) is prevented from slipping out from the rotation support part (34) in the distal end direction, by the locking claw (34a) and the ring-shaped locking protrusion (28c).

10. The syringe pressurization device (10) according to claim 8, wherein, in a state in which the pressing part (28) is accommodated deepest with respect to the accommodation part (48), a distal end surface of the pressing part (28) is located proximal of the most distal end position of the accommodation part (48).

11. A syringe system (11) comprising:
a prefilled syringe (12) including a barrel (14), a gasket (16) disposed to be slidable in the barrel (14), and a drug solution (M) filled in a liquid chamber formed by the barrel (14) and the gasket (16); and
a syringe pressurization device (10) attachable to the prefilled syringe (12),
wherein the syringe pressurization device (10) is the syringe pressurization device (10) according to any of the preceding claims.

12. The syringe system (11) according to claim 11, wherein the pressing part (28) is rotatable about an axis of the plunger rod (24) with respect to the plunger rod (24), and
frictional resistance between the pressing part (28) and the gasket (16) generated when the pressing part (28) rotates about the axis of the plunger rod (24) with respect to the gasket (16) is greater than frictional resistance between the pressing part (28) and the plunger rod (24) generated when the pressing part (28) rotates about the axis of the plunger rod (24) with respect to the plunger rod (24).

## Patentansprüche

1. Spritzen-Druckbeaufschlagungsvorrichtung (10), die so konfiguriert ist, dass sie an einer Spritze (12) angebracht werden kann, die einen Zylinder (14) und eine Dichtung (16) aufweist, die im Inneren des Zylinders (14) gleitet, wobei die Spritzen-Druckbeaufschlagungsvorrichtung (10) umfasst:
einen Kolben (20), der so konfiguriert ist, dass er die Dichtung (16) in Richtung eines distalen Endes vorschiebt; und
einen Griff (22), der an dem Zylinder (14) aus einer Richtung senkrecht zu einer Achse des Zylinders (14) anbringbar ist und den Kolben (20) trägt,
wobei der Kolben (20) eine Kolbenstange (24) umfasst, die ein Schraubengewinde (25) aufweist, das an einer äußeren Umfangsfläche davon angeordnet ist, und ein Druckteil (28), das an einem distalen Endabschnitt der Kolbenstange (24) angeordnet und so konfiguriert ist, dass es die Dichtung (16) in Richtung eines distalen Endes drückt,
wobei der Kolben (20) ein Spritzenkolben (20) ist, umfassend:
ein Drehbetätigungsteil (26), das an einem proximalen Endabschnitt der Kolbenstange (24) angeordnet ist,
wobei das Drehbetätigungsteil (26) ein Fingerhaken-Drehbetätigungsteil (26a), das in einer Richtung senkrecht zu einer Achse der Kolbenstange (24) vorsteht, und ein Einklemm-Drehbetätigungsteil (26b) umfasst, das in Richtung eines proximalen Endes von dem Fingerhaken-Drehbetätigungsteil (26a) koaxial zu der Kolbenstange (24) vorsteht,
das Schraubengewinde (25) konfiguriert ist, um mit einem Innengewindeteil (46) verschraubt zu werden,
der Spritzenkolben (20) so konfiguriert ist, dass er durch eine Schraubbewegung zwischen dem Schraubengewinde (25) und dem Innengewindeteil (46) in Richtung eines distalen Endes vorrückt, um die Dichtung (16) in Richtung eines distalen Endes zu drücken, und
ein Außendurchmesser des Einklemm-Drehbetätigungsteils (26b) kleiner ist als ein Außendurchmesser des Fingerhaken-Drehbetätigungsteils (26a), und wobei
der Griff (22) umfasst:
ein Flanschbefestigungsteil (44), das an einem Flansch befestigt werden kann, der an einem proximalen Endabschnitt des Zylinders (14) angeordnet ist,
ein Innengewindeteil (46), das proximal von dem Flanschbefestigungsteil (44) angeordnet und mit dem Schraubengewinde (25) verschraubt ist, und
ein Aufnahmeteil (48), das zwischen dem Flanschbefestigungsteil (44) und dem Innengewindeteil (46) angeordnet und so konfiguriert ist, dass es das Druckteil (28) aufnimmt.

2. Spritzen-Druckbeaufschlagungsvorrichtung (10) nach Anspruch 1, wobei in dem Spritzenkolben (20) das Fingerhaken-Drehbetätigungsteil (26a) eher als das Schraubengewinde (25) in einer Richtung senkrecht zur Achse der Kolbenstange (24) hervorsteht.

3. Spritzen-Druckbeaufschlagungsvorrichtung (10) nach Anspruch 1 oder 2, wobei bei dem Spritzenkolben (20) der Außendurchmesser des Einklemm-Drehbetätigungsteils (26b) kleiner ist als der Außendurchmesser des Schraubengewindes (25).

4. Spritzen-Druckbeaufschlagungsvorrichtung (10) nach einem der Ansprüche 1 bis 3, wobei in dem Spritzenkolben (20) das Einklemm-Drehbetätigungsteil (26b) eine zylindrische Form aufweist.

5. Spritzen-Druckbeaufschlagungsvorrichtung (10) nach Anspruch 4, wobei in dem Spritzenkolben (20) eine rutschhemmende Struktur für einen Finger an einem äußeren Umfangsabschnitt des Einklemm-Drehbetätigungsteils (26b) angeordnet ist.

6. Spritzen-Druckbeaufschlagungsvorrichtung (10) nach einem der Ansprüche 1 bis 5, wobei in dem Spritzenkolben (20) das Fingerhaken-Drehbetätigungsteil (26a) ein Paar von stabförmigen Fingerhakenteilen (27) aufweist, die in entgegengesetzten Richtungen zueinander von der Kolbenstange (24) hervorstehen.

7. Spritzen-Druckbeaufschlagungsvorrichtung (10) nach Anspruch 6, wobei in dem Spritzenkolben (20) ein verengter Teil (27a) und ein vorgewölbter Teil (27b), der außerhalb des verengten Teils (27a) angeordnet ist, in dem Paar von stabförmigen Fingerhakenteilen (27) angeordnet sind.

8. Spritzen-Druckbeaufschlagung (10) nach einem der vorhergehenden Ansprüche, wobei die Kolbenstange (24) an einem distalen Endabschnitt davon ein Drehunterstützungsteil (34) aufweist,
das Druckteil (28) ein scheibenförmiges Element ist, das auf dem Drehunterstützungsteil (34) der Kolbenstange (24) unterstützt ist, um um die Achse der Kolbenstange (24) drehbar zu sein und einen größeren Durchmesser als denjenigen der Kolbenstange (24) aufweist, und
das Aufnahmeteil (48) eine kreisförmige Nut ist, die in der Richtung des proximalen Endes in Bezug auf das Flanschbefestigungsteil (44) ausgespart ist und einen Innendurchmesser aufweist, der größer ist als ein Außendurchmesser des Druckteils (28).

9. Spritzen-Druckbeaufschlagungsvorrichtung (10) nach Anspruch 8, wobei das Drehunterstützungsteil (34) eine Verriegelungsklaue (34a) aufweist, die an einem distalen Ende davon nach außen vorsteht,
das Druckteil (28) ein rohrförmiges Basisteil (28a), das eine mittlere Seite davon umfasst, und ein ringförmiges Anlageteil (28b) aufweist, das sich von dem rohrförmigen Basisteil (28a) nach außen erstreckt und so konfiguriert ist, dass es an der proximalen Endfläche der Dichtung (16) anliegt,
ein ringförmiger Verriegelungsvorsprung (28c), der nach innen vorsteht, an einer inneren Umfangsfläche des rohrförmigen Basisteils (28a) angeordnet ist, und
das Druckteil (28) durch die Verriegelungsklaue (34a) und den ringförmigen Verriegelungsvorsprung (28c) an einem Herausrutschen aus dem Drehunterstützungsteil (34) in Richtung des distalen Endes gehindert wird.

10. Spritzen-Druckbeaufschlagungsvorrichtung (10) nach Anspruch 8, wobei in einem Zustand, in dem das Druckteil (28) am tiefsten in Bezug auf das Aufnahmeteil (48) aufgenommen ist, eine distale Endfläche des Druckteils (28) proximal von der am weitesten distal gelegenen Endposition des Aufnahmeteils (48) angeordnet ist.

11. Spritzensystem (11), umfassend:
eine vorgefüllte Spritze (12), die einen Zylinder (14), eine Dichtung (16), die so angeordnet ist, dass sie in dem Zylinder (14) verschiebbar ist, und eine Arzneimittellösung (M), die in eine durch den Zylinder (14) und die Dichtung (16) gebildete Flüssigkeitskammer gefüllt ist, umfasst; und
eine Spritzen-Druckbeaufschlagungsvorrichtung (10), die an der vorgefüllten Spritze (12) angebracht werden kann,
wobei die Spritzen-Druckbeaufschlagungsvorrichtung (10) die Spritzen-Druckbeaufschlagungsvorrichtung (10) nach einem der vorhergehenden Ansprüche ist.

12. Spritzensystem (11) nach Anspruch 11, wobei das Druckteil (28) um eine Achse der Kolbenstange (24) in Bezug auf die Kolbenstange (24) drehbar ist, und
der Reibungswiderstand zwischen dem Druckteil (28) und der Dichtung (16), der erzeugt wird, wenn sich das Druckteil (28) um die Achse der Kolbenstange (24) in Bezug auf die Dichtung (16) dreht, größer ist als der Reibungswiderstand zwischen dem Druckteil (28) und der Kolbenstange (24), der erzeugt wird, wenn sich das Druckteil (28) um die Achse der Kolbenstange (24) in Bezug auf die Kolbenstange (24) dreht.

## Revendications

1. Dispositif de mise sous pression de seringue (10) configuré pour être fixé à une seringue (12) comprenant un cylindre (14) et un joint (16) coulissant à l'intérieur du cylindre (14), le dispositif de mise sous pression de seringue (10) comprenant :
un piston (20) configuré pour faire progresser le joint (16) dans une direction d'extrémité distale ; et
une prise (22) pouvant être fixée au cylindre (14) depuis une direction perpendiculaire à un axe du cylindre (14) et supportant le piston (20),
dans lequel le piston (20) inclut une tige de piston (24) incluant un filetage de vis (25) disposé sur une surface circonférentielle externe de celui-ci, et une partie de pression (28) disposée au niveau d'une portion d'extrémité distale de la tige de piston (24) et configurée pour presser le joint (16) dans une direction d'extrémité distale,
dans lequel le piston (20) est un piston de seringue (20) comprenant :
une partie d'actionnement rotative (26) disposée au niveau d'une portion d'extrémité proximale de la tige de piston (24),
dans lequel la partie d'actionnement rotative (26) inclut une partie d'actionnement rotative de crochet pour doigt (26a) faisant saillie dans une direction perpendiculaire à un axe de la tige de piston (24), et une partie d'actionnement rotative à pincer (26b) faisant saillie dans une direction d'extrémité proximale à partir de la partie d'actionnement rotative de crochet pour doigt (26a) coaxialement avec la tige de piston (24),
le filetage de vis (25) est configuré pour être vissé avec une partie de vissage femelle (46),
le piston de seringue (20) est configuré pour progresser dans une direction d'extrémité distale par une action de vissage entre le filetage de vis (25) et la partie de vissage femelle (46) de manière à presser le joint (16) dans une direction d'extrémité distale, et
un diamètre externe de la partie d'actionnement rotative à pincer (26b) est plus petit qu'un diamètre externe de la partie d'actionnement rotative de crochet pour doigt (26a), et dans lequel
ladite prise (22) inclut :
une partie de fixation de bride (44) pouvant être fixée à une bride disposée au niveau d'une portion d'extrémité proximale du cylindre (14),
une partie de vissage femelle (46) disposée à proximité de la partie de fixation de bride (44) et vissée au filetage de vis (25), et
une partie de réception (48) disposée entre la partie de fixation de bride (44) et la partie de vissage femelle (46) et configurée pour recevoir la partie de pression (28).

2. Dispositif de mise sous pression de seringue (10) selon la revendication 1 dans lequel dans ledit piston de seringue (20) la partie d'actionnement rotative de crochet pour doigt (26a) fait saillie plutôt que le filetage de vis (25) dans une direction perpendiculaire à l'axe de la tige de piston (24).

3. Dispositif de mise sous pression de seringue (10) selon la revendication 1 ou 2, dans lequel dans ledit piston de seringue (20) le diamètre externe de la partie d'actionnement rotative à pincer (26b) est plus petit que le diamètre externe du filetage de vis (25).

4. Dispositif de mise sous pression de seringue (10) selon l'une quelconque des revendications 1 à 3, dans lequel dans ledit piston de seringue (20) la partie d'actionnement rotative à pincer (26b) a une forme cylindrique.

5. Dispositif de mise sous pression de seringue (10) selon la revendication 4, dans lequel dans ledit piston de seringue (20) une structure antidérapante pour un doigt est disposée sur une portion circonférentielle externe de la partie d'actionnement rotative à pincer (26b).

6. Dispositif de mise sous pression de seringue (10) selon l'une quelconque des revendications 1 à 5, dans lequel dans ledit piston de seringue (20) la partie d'actionnement rotative de crochet pour doigt (26a) inclut une paire de parties de crochet pour doigt de type tige (27) faisant saillie dans des directions opposées l'une à l'autre à partir de la tige de piston (24).

7. Dispositif de mise sous pression de seringue (10) selon la revendication 6, dans lequel dans ledit piston de seringue (20) une partie resserrée (27a), et une partie de renflement (27b) disposée à l'extérieur de la partie resserrée (27a) sont disposées dans la paire de parties de crochet pour doigt de type tige (27).

8. Dispositif de mise sous pression de seringue (10) selon l'une quelconque des revendications précédentes, dans lequel la tige de piston (24) inclut une partie de support de rotation (34) au niveau d'une portion d'extrémité distale de celui-ci,
la partie de pression (28) est un élément en forme de disque supporté sur la partie de support de rotation (34) de la tige de piston (24) pour être rotatif autour de l'axe de la tige de piston (24) et ayant un diamètre plus grand que celui de la tige de piston (24), et
la partie de réception (48) est un sillon circulaire encastré dans la direction d'extrémité proximale par rapport à la partie de fixation de bride (44) et ayant un diamètre interne plus grand qu'un diamètre externe de la partie de pression (28).

9. Dispositif de mise sous pression de seringue (10) selon la revendication 8, dans lequel la partie de support de rotation (34) inclut une griffe de verrouillage (34a) faisant saillie vers l'extérieur au niveau d'une extrémité distale de celle-ci,
la partie de pression (28) inclut une partie base tubulaire (28a) comprenant un côté central de celle-ci, et une partie de butée de forme annulaire (28b) s'étendant vers l'extérieur à partir de la partie base tubulaire (28a) et configurée pour buter contre la surface d'extrémité proximale du joint (16),
une saillie de verrouillage de forme annulaire (28c) faisant saillie vers l'intérieur est disposée sur une surface circonférentielle de la partie base tubulaire (28a), et
la griffe de verrouillage (34a) et la saillie de verrouillage de forme annulaire (28c) empêchent la partie de pression (28) de glisser hors de la partie de support de rotation (34) dans la direction d'extrémité distale.

10. Dispositif de mise sous pression de seringue (10) selon la revendication 8, dans lequel, dans un état dans lequel la partie de pression (28) est reçue plus profondément par rapport à la partie de réception (48), une surface d'extrémité distale de la partie de pression (28) se trouve à proximité de la position d'extrémité la plus distale de la partie de réception (48).

11. Système de seringue (11) comprenant :
une seringue préremplie (12) comprenant un cylindre (14),
un joint (16) disposé pour pouvoir coulisser dans le cylindre (14), et une solution de médicaments (M) remplie dans une chambre de liquide formée par le cylindre (14) et le joint (16) ; et
un dispositif de mise sous pression de seringue (10) pouvant être fixé à la seringue préremplie (12),
dans lequel le dispositif de mise sous pression de seringue (10) est le dispositif de mise sous pression de seringue (10) selon l'une quelconque des revendications précédentes.

12. Système de seringue (11) selon la revendication 11, dans lequel la partie de pression (28) peut pivoter autour d'un axe de la tige de piston (24) par rapport à la tige de piston (24), et
une résistance à la friction entre la partie de pression (28) et le joint (16) générée lorsque la partie de pression (28) pivote autour de l'axe de la tige de piston (24) par rapport au joint (16) est supérieure à une résistance à la friction entre la partie de pression (28) et la tige de piston (24) générée lorsque la partie de pression (28) pivote autour de l'axe de la tige de piston (24) par rapport à la tige de piston (24).
